# EUROPÄISCHE PATENTSCHRIFT

(11) **EP 2 664 349 B1**
(45) Veröffentlichungstag und Bekanntmachung des Hinweises auf die Patenterteilung: **03.02.2016**
(21) Anmeldenummer: 12003855.9
(22) Anmeldetag: 16.05.2012
(51) Int. Cl.: A61L 24/06

(54) **Pastenförmiger Knochenzement**
Bone cement in paste form
Ciment osseux pâteux

(43) Veröffentlichungstag der Anmeldung: 20.11.2013
(73) Patentinhaber: Heraeus Medical GmbH, 61273 Wehrheim (DE)
(72) Erfinder: Vogt, Sebastian, Dr., 99092 Erfurt (DE)
(74) Vertreter: Brand, Normen

(56) Entgegenhaltungen:
- WO-A1-2005/087850
- DE-A1- 3 320 918
- DE-A1- 19 501 933
- DE-A1-102010 055 759

## Beschreibung

Die vorliegende Erfindung betrifft einen Kit, die Verwendung eines Kits zur Herstellung einer Paste zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie sowie einen Formkörper.

Konventionelle Polymethylmethacrylat-Knochenzemente (PMMA-Knochenze-mente) sind seit Jahrzehnten bekannt und gehen auf die grundlegenden Arbeiten von Sir Charnley zurück (Chamley, J.: "Anchorage of the femoral head prosthesis of the shaft of the femur"; J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, (ii) einen Röntgenopaker und (iii) einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen. Ferner muss das Vermischen der Komponenten zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4,015,945, EP-A-0 674 888 und JP 2003-181270 offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Als Alternative zu den konventionellen Pulver-Flüssigkeits-Polymethylmeth-acrylatknochenzementen wurden pastenförmige Polymethylmethacrylatknochenzemente in den Offenlegungsschriften DE-A-10 2007 052 116, DE-A-10 2007 050 762, DE-A-10 2007 050 763 und DE101020055759 beschrieben. Dabei werden die Knochenzemente als vorgemischte, lagerstabile Pasten dem Anwender zur Verfügung gestellt. Diese Pasten enthalten jeweils ein radikalisch polymerisierbares Methacrylatmonomer, ein in diesem Methacrylatmonomer lösliches Polymer und ein in diesem Methacrylatmonomer unlösliches partikuläres Polymer (da beide Pasten ein unlösliches partikuläres Polymer beinhalten, werden solche Systeme als "symmetrisch" bezeichnet). In einer dieser Pasten ist zudem ein radikalischer Polymerisationsinitiator enthalten, während die andere Paste einen Polymerisationsaktivator aufweist. Der aus diesen Pasten hergestellte Knochenzement weist als Folge der gewählten Zusammensetzung eine ausreichend hohe Viskosität und Kohäsion auf, um dem Blutungsdruck bis zur Aushärtung standzuhalten. Beim Vermischen der beiden Pasten reagiert der Polymerisationsinitiator mit dem Akzelerator unter Bildung von Radikalen, die die radikalische Polymerisation der Methacrylatmonomere initiieren.

In den aufgeführten Schriften werden als Initiatorsysteme Barbiturate in Kombination mit Schwermetall-Ionen und Halogenid-Ionen vorgeschlagen. Weiterhin wird das Redox-InitiatorSystem Dibenzoylperoxid/tertiäres aromatisches Amin erwähnt.

Im EP 0 659 859 A1 wird ein Klebstoff offenbart, der in einer ersten Komponente neben Monomeren ein Hydroperoxid und in einer zweiten Komponente neben Monomeren ein Schwermetallsalz und ein Sulfimid enthält. Die Verwendung von tertiären Aminen als Co-Beschleuniger wird nicht erwähnt.

Eine zweiteilige Klebstoffzusammensetzung wird auch in DE 33 20 918 A1 offenbart. Der eine Teil des Klebstoffs enthält ein polymerisierbares Acrylat oder Methacrylat, ein Peroxid, gegebenenfalls ein Sulfimid und gegebenenfalls ein Hydrazinderivat. Der zweite Teil des Klebstoffs enthält neben einem polymerisierbaren Acrylat oder Methacrylat, ein Übergangsmetallsalz und gegebenenfalls ein Amin als Co-Beschleuniger. Eigene Versuche zeigten, dass Gemische aus Methacrylat-Monomer (Ethylenglykoldimethacrylat), Cumol-hydroperoxid und Saccharin (Sulfimid) bei erhöhter Temperatur (50 °C) innerhalb weniger Tage bei ihrer Vermischung mit bei Raumtemperatur gelagerten Gemischen aus Methacrylat-Monomer, darin gelöstem Kupfer(II)-2-ethylhexanoat und N,N-Dimethyl-p-toluidin hinsichtlich des Einsetzens der exothermen Polymerisation nachlassen. Dieser Effekt ist offensichtlich auf eine oxidative Zersetzung des Saccharins durch Einwirkung des Cumol-hydroperoxids zurückzuführen.

In DE 195 01 933 A1 wird ein aerob aushärtbarer Klebstoff auf Basis von Methacrylat-Monomeren mit einem Siedepunkt größer 120 °C beschrieben. Dieser 2-Komponenten-Klebstoff enthält in einer Paste ein Hydroperoxid und in der zweiten Paste mindestens eine Schwermetallverbindung. Als Beschleuniger werden Sulfimide, tertiäre Amine und Hydrazinderivate vorgeschlagen. Der besondere Vorteil dieser Klebstoffe soll darin bestehen, dass auf Grund der Verwendung von Methacrylat-Monomeren mit einem Siedepunkt größer 120 °C an der Grenzfläche zur Luft keine klebrige Schicht (Dispersionsschicht) erhalten wird. Die Dispersionsschicht wird durch Störung der Polymerisation infolge der Einwirkung von Luftsauerstoff gebildet und enthält neben Oligomeren und Polymeren auch nicht umgesetzte Monomere. Dadurch können Dispersionsschichten bei Kontakt mit humanem Gewebe Verträglichkeitsprobleme verursachen.

Der vorliegenden Erfindung lag die Aufgabe zugrunde, die sich aus dem Stand der Technik ergebenden Nachteile im Zusammenhang mit auf mindestens zwei Pasten basierenden Knochenzement-Systemen zu überwinden.

Insbesondere lag der vorliegenden Erfindung die Aufgabe zugrunde, einen Polymethylmethacrylat-Knochenzement-Kit auf der Basis zweier radikalisch polymerisierbarer Pasten bereitzustellen, wobei sich die Pasten, solange sie getrennt voneinander vorliegen, durch eine möglichst hohe Polymerisationsstabilität auszeichnen (also möglichst nicht zur Spontanpolymerisation neigen). Nach dem Vermischen der beiden Pasten soll eine Paste entstehen, die innerhalb weniger Minuten selbsttätig durch radikalische Polymerisation aushärtet. Die Geschwindigkeit, mit der nach dem Vermischen der beiden Pasten die radikalische Polymerisation erfolgt, soll dabei möglichst gleichbleibend und insbesondere unabhängig davon sein, wie lange die Pasten zuvor getrennt voneinander gelagert wurden.

Der vorliegenden Erfindung lag auch die Aufgabe zugrunde, einen Polymethylmethacrylat-Knochenzement-Kit auf der Basis zweier radikalisch polymerisierbarer Pasten bereitzustellen, welche nach dem Vermischen innerhalb weniger Minuten selbsttätig durch radikalische Polymerisation und Erhalt eines Polymerisates aushärten, das auf seiner Oberfläche möglichst keine fühlbar klebrige Dispersionsschicht aufweist.

Einen Beitrag zur Lösung der eingangs genannten Aufgaben leistet ein Knochenzement - Kit, aufweisend eine Paste A und eine Paste B,
wobei
(a) Paste A
   (a1) wenigstens ein radikalisch polymerisierbares Monomer mit einem Siedepunkt von weniger als 120°C bei einem Druck von 1.013 mbar,
   (a2) als Polymerisationsinitiator wenigstens ein Peroxid, und
   (a3) als Co-Polymerisationsbeschleuniger wenigstens ein tertiäres Amin, wenigstens ein Amidin oder eine Mischung aus wenigstens einem tertiärem Amin und wenigstens einem Amidin, und
(b) Paste B
   (b1) wenigstens ein radikalisch polymerisierbares Monomer mit einem Siedepunkt von weniger als 120°C bei einem Druck von 1.013 mbar,
   (b2) als Polymerisationsbeschleuniger wenigstens eine Schwermetallverbindung, und
   (b3) als Co-Polymerisationsbeschleuniger wenigstens ein Sulfimid, wenigstens ein Dicarbonsäureimid oder eine Mischung aus wenigstens einem Sulfimid und einem Dicarbonsäureimid,
beinhalten, wobei mindestens eine der Pasten A und B als Komponente (a4) bzw. (b4) wenigstens einen in (a1) bzw. (b1) unlöslichen Füllstoff beinhaltet.

Die Erfindung beruht unter anderem auf dem überraschenden Befund, dass sich an der Oberfläche klebfreie Polymerisate aus Pasten mit dem Initiatorsystem Peroxid/Sulfimid/tertiäres Amin auch bei Verwendung von Methacrylat-Monomeren mit einem Siedepunkt kleiner 120 °C, im Gegensatz zur Lehre der DE 195 01 933 A2, herstellen lassen. Dadurch sind diese Polymerisate bei Verwendung von geeigneten Monomeren auch im Kontakt mit humanem Gewebe verträglich.

Erfindungsgemäß wird unter einem "Kit" ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Paste A beinhaltet als Komponente (a1) ein radikalisch polymerisierbares Monomer mit einem Siedepunkt von weniger als 120°C bei einem Druck von 1.013 mbar, wobei es sich vorzugsweise um ein Monomer handelt, welches bei einer Temperatur von 25°C und einem Druck von 1.013 mbar flüssig ist.

Das radikalisch polymerisierbare Monomer (a1) ist vorzugsweise ein Methacrylat-Monomer, insbesondere ein Methacrylsäureester. Vorzugsweise handelt es sich bei dem Methacrylsäureester (a1) um einen monofunktionellen Methacrylsäureester. Dieser ist vorzugsweise hydrophob. Durch die Verwendung von hydrophoben monofunktionellen Methacrylsäureestern (a1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem radikalisch polymerisierbaren Monomer (a1) um Methacrylsäuremethylester, Methacrylsäureethylester oder um eine Mischung aus diesen beiden Monomeren.

Das erfindungsgemäß eingesetzte radikalisch polymerisierbare Monomer (a1) weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer (a1) zeichnet sich vorzugsweise dadurch aus, dass eine wässrige Lösung des radikalisch polymerisierbaren Monomers (a1) einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Paste A enthält vorzugsweise 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste A, des wenigstens einen radikalisch polymerisierbaren Monomers (a1).

Paste A beinhaltet weiterhin als Komponente (a2) wenigstens ein Peroxid, welches vorzugsweise eine Halbwertszeit > 10 Stunden, besonders bevorzugt > 24 Stunden und noch mehr bevorzugt > 48 Stunden, jeweils gemessen bei 70°C, besitzt. Bei der Bestimmung der Halbwertszeit wird der Peroxid-Gehalt durch Iod-Titration bestimmt, bei der Iodid-Ionen durch das Peroxid zu Iod oxidiert werden.

Besonders bevorzugt ist das Peroxid (a2) ausgewählt aus der Gruppe bestehend Cumolhydroperoxid, 1,1,3,3-Tetramethylbutyl-hydroperoxid, t-Butyl-hydro-peroxid, t-Amyl-hydroperoxid, Di-Isopropylbenzen-mono-hydroperoxid, Di-t-butyl-peroxid, Dicumylperoxid und t-Butylcumyl-peroxid.

Die Paste A beinhaltet das wenigstens eine Peroxid (a2) vorzugsweise in einer Menge ein einem Bereich von 0,01 bis 10 Gew.-%, mehr bevorzugt in einem Bereich von 0,1 bis 8 Gew.-% und noch mehr bevorzugt in einem Bereich von 1 bis 5 Gew.-%, jeweils bezogen auf das Gesamtgewicht der Paste A.

Paste A beinhaltet weiterhin als Komponente (a3) wenigstens ein tertiäres Amin, wenigstens ein Amidin oder eine Mischung aus wenigstens einem tertiärem Amin und wenigstens einem Amidin als Co-Polymerisationsbeschleuniger.

Bei dem tertiären Amin handelt es sich vorzugsweise um ein Amin ausgewählt aus der Gruppe bestehend aus Tributylamin, Triethanolamin, N,N-Dimethyl-p-toluidin, N,N-Bis(2-hydroxy-ethyl)-p-toluidin und N,N-Dimethylanilin handelt. Besonders vorteilhaft ist die Kombination von N,N-Dimethyl-p-toluidin und N,N-Bis(2-hydroxyethyl)-p-toluidin. Mit dieser Kombination lässt sich das Aushärtungsverhalten der gemischten Paste C durch Variation des Verhältnisses von N,N-Dimethyl-p-toluidin zu N,N-Bis(2-hydroxyethyl)-p-toluidin sehr gut steuern.

Anstelle des tertiären Amins (oder in Kombination mit dem tertiären Amin) kann auch ein Amidin als Base verwendet werden. Dabei sind besonders 1,8-Diazabicyclo[5.4.0]undec-7-en (DBU) und 1,5-Diazabicyclo(4.3.0)non-5-en (DBN) bevorzugt.

Die Paste A beinhaltet den Co-Polymerisationsbeschleuniger (a3) vorzugsweise in einer Menge ein einem Bereich von 0,1 bis 20 Gew.-%, mehr bevorzugt in einem Bereich von 0,5 bis 10 Gew.-% und noch mehr bevorzugt in einem Bereich von 1 bis 5 Gew.-%, jeweils bezogen auf das Gesamtgewicht der Paste A.

Paste B beinhaltet als Komponente (b1) ebenfalls ein radikalisch polymerisierbares Monomer mit einem Siedepunkt von weniger als 120°C bei einem Druck von 1.013 mbar, wobei es sich vorzugsweise um ein Monomer handelt, welches bei einer Temperatur von 25°C und einem Druck von 1.013 mbar flüssig ist. Das radikalisch polymerisierbare Monomer (b1) kann in einem Kit identisch sein von dem radikalisch polymerisierbaren Monomer (a1) oder sich von diesem unterscheiden, wobei es bevorzugt ist, dass das radikalisch polymerisierbare Monomer (a1) und das radikalisch polymerisierbare Monomer (b1) identisch sind.

Das radikalisch polymerisierbare Monomer (b1) ist vorzugsweise ein Methacrylat-Monomer, insbesondere ein Methacrylsäureester. Vorzugsweise handelt es sich bei dem Methacrylsäureester (b1) um einen monofunktionellen Methacrylsäureester. Dieser ist vorzugsweise hydrophob.

Durch die Verwendung von hydrophoben monofunktionellen Methacrylsäureestern (b1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem radikalisch polymerisierbaren Monomer (b1) um Methacrylsäuremethylester, Methacrylsäureethylester oder um eine Mischung aus diesen beiden Monomeren.

Gemäß einer weiteren bevorzugten Ausführungsform handelt es sich bei dem radikalisch polymerisierbaren Monomer (b1) nicht um einen von Bisphenol A abgeleiteten Methacrylsäureester.

Das erfindungsgemäß eingesetzte radikalisch polymerisierbare Monomer (b1) weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer (b1) zeichnet sich vorzugsweise dadurch aus, dass eine wässrige Lösung des radikalisch polymerisierbaren Monomers (b1) einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Paste B enthält vorzugsweise 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste B, des wenigstens einen radikalisch polymerisierbaren Monomers (b1).

Paste B beinhaltet weiterhin als Komponente (b2) wenigstens Schwermetallverbindung als Polymerisationsbeschleuniger, wobei die Schwermetallverbindung ein Schwermetallsalz oder ein Schwermetallkomplex sein kann. Gemäß einer besonders bevorzugten Ausführungsform handelt es sich bei den Schwermetallverbindungen (b2) um Verbindungen von Metallen, die ihre Oxidationsstufe wechseln können. In diesem Zusammenhang erfindungsgemäß bevorzugt sind Verbindungen von Kupfer(II), Eise (II), Eisen(III), Mangan(II), Mangan(III), Cobalt(II) und Cobalt(III), wobei Kupfer(II)-Verbindungen ganz besonders bevorzugt sind. In diesem Zusammenhang besonders bevorzugte Schwermetallverbindungen (b2) sind ausgewählt aus der Gruppe bestehend aus Kupfer(II)-hydroxid, Kupfer(II)metharylat, Kupfer(II)acetylacetonat, Kupfer(II)-2-ethyl-hexanoat, Cobalt(II)-hydroxid, Cobalt(II)-2-ethyl-hexanoat und basischem Kupfer(II)-carbonat.

Die Paste B beinhaltet die Schwermetallverbindung (b2) vorzugsweise in einer Menge in einem Bereich von 0,0005 bis 0,5 Gew.-%, mehr bevorzugt in einem Bereich von 0,001 bis 0,05 Gew.-% und ganz besonders bevorzugt in einem Bereich von 0,001 bis 0,01 Gew.-%, jeweils bezogen auf das Gesamtgewicht der Paste B.

Paste B beinhaltet weiterhin als Komponente (b3) wenigstens ein Sulfimid, wenigstens ein Dicarbonsäureimid oder eine Mischung aus wenigstens einem Sulfimid und wenigstens einem Dicarbonsäureimid als Co-Polymerisations-beschleuniger, wobei Saccharin ein besonders bevorzugtes Sulfimid und Phthalimid, Maleimid und Succinimid besonders bevorzugte Dicarbonsäureimide sind. Daneben ist auch die Verwendung von Tetracarbonsäurediimiden wie Pyromellithsäurediimid bevorzugt.

Die Paste B beinhaltet die Co-Polymerisationsbeschleuniger (b3) vorzugsweise in einer Menge in einem Bereich von 0,1 bis 10 Gew.-%, mehr bevorzugt in einem Bereich von 0,5 bis 8 Gew.-% und ganz besonders bevorzugt in einem Bereich von 1 bis 5 Gew.-%, jeweils bezogen auf das Gesamtgewicht der Paste B.

Der erfindungsgemäße Kit ist weiterhin dadurch gekennzeichnet, dass mindestens eine der Pasten A und B als Komponente (a4) bzw. (b4) wenigstens einen in (a1) bzw. (b1) unlöslichen Füllstoff beinhaltet. Sofern eine der beiden Pasten einen unlöslichen Füllstoff beinhaltet und die andere Paste überhaupt keinen unlöslichen Füllstoff oder einen unlöslichen Füllstoff in einer im Vergleich zur Menge in der anderen Paste vernachlässigbaren Menge, so handelt es sich um einen sogenannten "asymmetrischen" Kit. Ist hingegen der unlösliche Füllstoff in beiden Pasten in etwa vergleichbarer Menge vorhanden, so handelt es sich um einen sogenannten "symmetrischen" Kit.

Bei dem Füllstoff (a4) (im Falle der Paste A) bzw. (b4) (im Falle der Paste B) handelt es sich um einen bei Raumtemperatur festen Stoff, der in der Lage ist, die Viskosität der aus den übrigen in Paste A bzw. Paste B enthaltenen Bestandteilen zusammengesetzten Mischung zu erhöhen. Der Füllstoff (a4) bzw. (b4) soll biokompatibel sein.

Gemäß einer bevorzugten Ausführungsform ist der Füllstoff (a4) bzw. (b4) aus Polymeren, anorganischen Salzen, anorganischen Oxiden, Metallen und Metalllegierungen ausgewählt.

Der Füllstoff (a4) bzw. (b4) ist vorzugsweise partikulär. Gemäß einer besonders bevorzugten Ausführungsform weist der Füllstoff (a4) bzw. (b4) eine durchschnittliche Teilchengröße im Bereich von 10 nm bis 100 µm und besonders bevorzugt im Bereich von 100 nm bis 10 µm auf. Unter durchschnittlicher Teilchengröße wird vorliegend ein Größenbereich verstanden, der von wenigstens 90 Prozent der Teilchen eingenommen wird.

Im Rahmen der Erfindung werden unter dem Begriff Polymere sowohl Homopolymere als auch Copolymere zusammengefasst.

Bei dem als Füllstoff (a4) bzw. (b4) verwendbaren Polymer handelt es sich vorzugsweise um ein Polymer mit einer gewichtsmittleren Molmasse von wenigstens 150.000 g/mol. Die Angabe der Molmasse bezieht sich hierin auf die viskosimetrisch bestimmte Molmasse. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat) und Poly(styren-co-methylmethacrylat) besteht. Das Polymer kann jedoch ebenso aus der Gruppe ausgewählt sein, die aus Polyethylen, Polypropylen oder Polybutadien besteht. Das Polymer kann zudem vernetzt oder unvernetzt sein, wobei vernetzte Polymere besonders bevorzugt sind. Die Vernetzung erfolgt dabei vorzugsweise mit einer difunktionellen Verbindung. Die difunktionelle Verbindung kann beispielsweise aus der Gruppe ausgewählt sein, die aus Alkylenglykoldimeth-acrylaten besteht. Als Vernetzter hat sich beispielsweise Ethylenglykol-dimethacrylat bewährt.

Das als Füllstoff (a4) bzw. (b4) verwendbare anorganische Salz kann ein im radikalisch polymerisierbaren Monomer (a1) bzw. (b1) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (a4) bzw. (b4) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (a4) bzw. (b4) verwendbaren Metall kann es sich beispielsweise um ein Übergangsmetall handeln. Gemäß einer bevorzugten Ausführungsform ist das Metall Tantal oder Wolfram.

Die als Füllstoff (a4) bzw. (b4) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Füllstoff (a4) bzw. (b4) ist in dem radikalisch polymerisierbaren Monomer (a1) bzw. (b1) unlöslich. Erfindungsgemäß ist der Füllstoff (a4) bzw. (b4) in dem radikalisch polymerisierbaren Monomer (a1) bzw. (b1) unlöslich, wenn der Füllstoff (a4) bzw. (b4) bei einer Temperatur von 25°C eine Löslichkeit im radikalisch polymerisierbaren Monomer (a1) bzw. (b1) von weniger als 50 g/l, vorzugsweise weniger als 25 g/l, mehr bevorzugt weniger als 10 g/l und noch mehr bevorzugt weniger als 5 g/l aufweist.

Erfindungsgemäß besonders bevorzugt ist es, dass das wenigstens eine in (a1) bzw. (b1) unlösliche Polymer ausgewählt ist aus der Gruppe bestehend aus vernetztem Poly(methylmethacrylatco-methylacrylat), vernetztem Poly(methylmeth-acrylat) und einer Mischung dieser beiden Polymere.

Weiterhin kann erfindungsgemäß die Paste A, die Paste B oder die Paste A und die Paste B, besonders bevorzugt jedoch die Paste A und die Paste B, ein in (a1) bzw. (b1) lösliches Polymer (a5) bzw. (b5) beinhalten. Dieses Polymer (a5) bzw. (b5) ist erfindungsgemäß in dem polymerisierbaren Monomer, das in der Paste enthalten ist, in der auch das lösliche Polymer enthalten ist, löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer (a1) bzw. (b1) lösliche Polymer (a5) bzw. (b5) kann ein Homopolymer oder ein Copolymer sein. Bei diesem Polymer (a5) bzw. (b5) handelt es sich vorzugsweise um ein Polymer mit einer gewichtsmittleren Molmasse von wenigstens 150.000 g/mol. Beispielsweise kann es sich bei dem Polymer (a5) bzw. (b5) um ein Polymer oder Copolymer eines Methacrylsäureesters handeln. Gemäß einer besonders bevorzugten Ausführungsform ist das wenigstens eine Polymer (a5) bzw. (b5) aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methyl-acrylat) und Poly(styren-co-methylmethacrylat) besteht.

Die Menge des in dem radikalisch polymerisierbaren Monomer (a1) bzw. (b1) löslichen Polymers (a5) bzw. (b5) in der Paste, die dieses Polymer enthält, hängt davon ab, ob die entsprechende Paste ein in dem radikalisch polymerisierbaren Monomer (a1) bzw. (b1) unlöslichen Füllstoff (a4) bzw. (b4) enthält. Üblicherweise liegt die Menge des in dem radikalisch polymerisierbaren Monomer (a1) bzw. (b1) löslichen Polymers (a5) bzw. (b5) in der Paste, die dieses Polymer enthält, in einem Bereich von 1 bis 85 Gew.-%, bezogen auf das Gesamtgewicht der Paste, welche dieses lösliche Polymer enthält.

Neben den vorstehend erläuterten Bestandteilen können die Pasten A und B weitere Bestandteile aufweisen. Diese weiteren Bestandteile können jeweils entweder in Paste A, in Paste B oder in der Paste A und der Paste B enthalten sein.

Gemäß einer bevorzugten Ausführungsform ist in wenigstens einer der Pasten A und B wenigstens ein Röntgenopaker enthalten. Beim Röntgenopaker kann es sich um einen fachüblichen Röntgenopaker handeln. Geeignete Röntgenopaker können in dem radikalisch polymerisierbaren Monomer (a1) oder dem radikalisch polymerisierbaren Monomer (b1) löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht.

Gemäß einer weiteren bevorzugten Ausführungsform ist in wenigstens einer der Pasten A und B wenigstens ein Farbstoff enthalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer (a1) oder in dem wenigstens einen radikalisch polymerisierbaren Monomer (a2) löslich oder unlöslich sein. Gemäß einer besonders bevorzugten Ausführungsform wird der Farbstoff aus der Gruppe ausgewählt, die aus E101, E104, E 132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform ist in wenigstens einer der Pasten A und B wenigstens ein pharmazeutischer Wirkstoff enthalten. Der wenigstens eine pharmazeutische Wirkstoff kann in wenigstens einer der Pasten A und B in gelöster oder suspendierter Form enthalten sein.

Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum.

Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycin-hydrochlorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht.

Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

Gemäß einer weiteren bevorzugten Ausführungsform ist in wenigstens einer der Pasten A und B wenigstens ein biokompatibles Elastomer enthalten. Das biokompatible Elastomer ist vorzugsweise partikulär. Vorzugsweise ist das biokompatible Elastomer in dem wenigstens einen radikalisch polymerisierbaren Monomer (a1) oder in dem wenigstens einen radikalisch polymerisierbaren Monomer (b1) löslich. Als besonders geeignet hat sich die Verwendung von Polybutadien als biokompatibles Elastomer erwiesen.

Gemäß einer weiteren bevorzugten Ausführungsform enthält wenigstens eine der Pasten A und B wenigstens ein Monomer mit Haftgruppen. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform ist in wenigstens einer der Pasten A und B wenigstens ein Stabilisator enthalten. Der Stabilisator soll geeignet sein, eine Spontanpolymerisation der in den Pasten A und B enthaltenen radikalisch polymerisierbaren Monomere (a1) bzw. (b1) zu verhindern. Ferner soll der Stabilisator keine störenden Wechselwirkungen mit den anderen in den Pasten enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Gemäß einer ersten besonderen Ausgestaltung des erfindungsgemäßen Kits handelt es sich bei dem Kit um einen "asymmetrischen" Kit. In diesem Zusammenhang ist es bevorzugt, dass die Paste A 20 bis 70 Gew.-%, besonders bevorzugt 25 bis 60 Gew.-%, noch mehr bevorzugt 30 bis 55 Gew.-% und am meisten bevorzugt 34 bis 47 Gew.-%, jeweils bezogen auf das Gesamtgewicht der Paste A, des in (a1) unlöslichen Füllstoffs (a4) und Paste B weniger als 5 Gew.-%, besonders bevorzugt weniger als 1 Gew.-%, noch mehr bevorzugt weniger als 0,1 Gew.-% und darüber hinaus bevorzugt weniger als 0,01 Gew.-%, jeweils bezogen auf das Gesamtgewicht der Paste B, des in (b1) unlöslichen Füllstoffs (b4) enthält, wobei es am meisten bevorzugt ist, dass die Paste B überhaupt kein in (b1) unlöslichen Füllstoff (b4) enthält.

Weiterhin ist es im Zusammenhang mit dieser ersten besonderen Ausgestaltung des erfindungsgemäßen Kits bevorzugt, dass Paste A ein in (a1) lösliches Polymer (a5) in einer Menge in einem Bereich von 1 bis 25 Gew.-%, besonders bevorzugt in einem Bereich von 2 bis 20 Gew.-%, noch mehr bevorzugt in einem Bereich von 2 bis 18 Gew.-% und am meisten bevorzugt in einem Bereich von 3 bis 16 Gew.-%, jeweils bezogen auf das Gesamtgewicht der Paste A, und Paste B ein in (b1) lösliches Polymer (b5) in einer Menge in einem Bereich von 25 bis 85 Gew.-%, besonders bevorzugt in einem Bereich von 35 bis 85 Gew.-%, noch mehr bevorzugt in einem Bereich von 40 bis 80 Gew.-% und am meisten bevorzugt in einem Bereich von 50 bis 75 Gew.-%, jeweils bezogen auf das Gesamtgewicht der Paste B, beinhalten.

Weiterhin ist es im Zusammenhang mit dieser ersten besonderen Ausgestaltung des erfindungsgemäßen Kits bevorzugt, dass das Gewichtsverhältnis von in (b1) unlöslichem Füllstoff (b4) zu dem wenigstens einen in (b1) löslichen Polymer (b5) höchstens 0,2, besonders bevorzugt höchstens 0,15, noch mehr bevorzugt höchstens 0,1, nicht mehr bevorzugt höchstens 0,05, besonders bevorzugt höchstens 0,02 und ganz besonders bevorzugt 0 beträgt.

Gemäß einer zweiten besonderen Ausgestaltung des erfindungsgemäßen Kits handelt es sich bei dem Kit um einen "symmetrischen" Kit. In diesem Zusammenhang ist es bevorzugt, dass die Paste A 15 bis 85 Gew.-%, besonders bevorzugt 15 bis 80 Gew.-% und noch mehr bevorzugt 20 bis 75 Gew.-%, jeweils bezogen auf das Gesamtgewicht der Paste A, des in (a1) unlöslichen Füllstoffs (a4) und Paste B 15 bis 85 Gew.-%, besonders bevorzugt 15 bis 80 Gew.-% und noch mehr bevorzugt 20 bis 75 Gew.-%, jeweils bezogen auf das Gesamtgewicht der Paste B, des in (b1) unlöslichen Füllstoffs (b4) beinhalten.

Weiterhin ist es im Zusammenhang mit dieser zweiten besonderen Ausgestaltung des erfindungsgemäßen Kits bevorzugt, dass Paste A ein in (a1) lösliches Polymer (a5) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, jeweils bezogen auf das Gesamtgewicht der Paste A, und/oder Paste B ein in (b1) lösliches Polymer (b5) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, bezogen auf das Gesamtgewicht der Paste B, beinhalten.

Erfindungsgemäß dient der Kit, der wenigstens die Pasten A und B enthält, zur Herstellung von Knochenzement.

Dazu werden die wenigstens zwei Pasten A und B miteinander vermischt, wobei wiederum eine Paste, Paste C, erhalten wird.

Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B.

Das Mischen kann mit üblichen Mischgeräten, beispielsweise einem statischen Mischer oder einem dynamischen Mischer erfolgen.

Das Mischen kann unter Vakuum erfolgen. Die Verwendung des erfindungsgemäßen Initiatorsystems erlaubt jedoch auch das vakuumfreie Mischen der Paste A und B ohne Beeinträchtigung der Eigenschaften des Knochenzements.

Nach dem Vermischen der Pasten des Kits ist die letztlich erhaltene Paste C nach der Norm ISO 5833 klebfrei und kann sofort verarbeitet werden.

Der aus der Paste C durch Aushärtung entstehende Knochenzement erreicht etwa 3 bis 15 Minuten nach dem Vermischen der im Kit enthaltenen Pasten eine hohe Festigkeit.

Der erfindungsgemäße Kit kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacern und zur Herstellung von Trägermaterialien für die lokale Antibiotika-Therapie verwendet werden.

Unter dem Begriff *"Spacer"* werden dabei erfindungsgemäß Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.

Die vorstehend beschriebenen erfindungsgemäßen Verwendungen von Knochenzement sind aus der Literatur bekannt und dort mannigfach beschrieben.

Erfindungsgemäß wird der Kit für die vorstehend beschrieben Verwendungen eingesetzt, indem vorzugsweise aus den im Kit enthaltenen Pasten durch Vermischen eine Paste hergestellt wird, die analog zu den aus dem Stand der Technik bekannten Pasten für die beschriebenen Verwendungen eingesetzt wird.

Einen Beitrag zur Lösung der eingangs genannten Aufgaben leistet auch ein Formkörper, erhalten durch die Polymerisation der erfindungsgemäßen polymerisierbaren Zusammensetzung oder durch die Polymerisation einer Paste, erhältlich durch das Vermischen der Paste A und der Paste B des erfindungsgemäßen Kits. Formkörper im Sinne der vorliegenden Erfindung können jedwede dreidimensionalen Körper sein, insbesondere die vorstehend beschriebenen "Spacer".

Die Erfindung wird durch die nachstehend beschriebenen Beispiele erläutert.

### BEISPIELE

Die Pasten A der Beispiele A1-21 wurden durch einfaches Vermischen der Komponenten hergestellt. Die gebildeten Pasten wurden dann über Nacht bei Raumtemperatur gelagert.

### Paste A

| | Zusammensetzung der Pasten A | | | | | | | | |
|---|---|---|---|---|---|---|---|---|---|
| Beispiel-Nr. | CH [g] | BH [g] | EG [g] | MA [g] | MMA [g] | PL 1 [g] | PL2 [g] | ZrO₂ [g] | Stab [mg] |
| A1 | 0,50 | 0,8 | 0,1 | 0,4 | 17,7 | 5,6 | 15,5 | 4,8 | 20 |
| A2 | 0,50 | 1,0 | 0,1 | 0,4 | 17,7 | 5,6 | 15,5 | 4,8 | 20 |
| A3 | 0,50 | 1,2 | 0,4 | 0,4 | 17,7 | 5,6 | 15,5 | 4,8 | 20 |
| A4 | 0,10 | 1,2 | 0,4 | 0,4 | 17,7 | 5,6 | 15,5 | 4,8 | 20 |
| A5 | 0,10 | 1,2 | 0,7 | 0,4 | 17,7 | 5,6 | 15,5 | 4,8 | 20 |
| A6 | 0,10 | 1,2 | 1,0 | 0,4 | 17,7 | 5,6 | 15,5 | 4,8 | 20 |
| A7 | 0,10 | 1,2 | 1,0 | 0,4 | 17,7 | 5,6 | 15,5 | 4,8 | 20 |
| A8 | 0,05 | 1,2 | 0,1 | 0,4 | 17,7 | 5,6 | 15,5 | 4,8 | 20 |
| A9 | 0,05 | 2,0 | 0,1 | 0,4 | 17,7 | 5,6 | 15,5 | 4,8 | 20 |
| A10 | 0,10 | 2,0 | 0,1 | 0,4 | 17,7 | 5,6 | 15,5 | 4,8 | 20 |
| A11 | 0,05 | 1,2 | 0,7 | 0,4 | 17,7 | 5,6 | 15,5 | 4,8 | 20 |
| A12 | 0,05 | 0,6 | 0,7 | 0,4 | 17,7 | 5,6 | 15,5 | 4,8 | 20 |
| A13 | 0,05 | 1,2 | 1,3 | 0,4 | 17,7 | 5,6 | 15,5 | 4,8 | 20 |
| A14 | 0,20 | 2,0 | 0,1 | 0,4 | 17,7 | 5,6 | 15,5 | 4,8 | 20 |

- CH:: Cumolhydroperoxid
- BH:: N,N-Bis-(2-hydroxyethyl)-p-toluidin
- EG:: Ethylenglykoldimethacrylat
- MA:: Methacrylamid
- MMA:: Methylmethacrylat
- PL1:: lineares Poly(methylmethacrylat-co-methylacrylat) Mw < 500.000 g/mol
- PL2:: partikuläres, unlösliches, vernetztes Polymethylmethacrylat
- ZrO₂:: Zirkoniumdioxid
- Stab:: 2,6-Di-t-butyl-4-methyl-phenol

### Paste A

| Beispiel Nr. | CH [g] | BH [g] | DM [g] | EG [g] | MA [g] | MMA [g] | PL1 [g] | PL2 [g] | ZrO₂ [g] | Stab [mg] |
|---|---|---|---|---|---|---|---|---|---|---|
| A15 | 0,0 5 | 1,6 | 0,4 | 0,1 | 0,4 | 17,7 | 5,6 | 15,5 | 4,8 | 20 |
| A16 | 0,0 5 | 0,8 | 0,4 | 0,1 | 0,4 | 17,7 | 5,6 | 15,5 | 4,8 | 20 |
| A17 | 0,1 0 | 1,6 | 0,4 | 0,1 | 0,4 | 17,7 | 5,6 | 15,5 | 4,8 | 20 |
| A18 | 0,0 5 | 1,4 | 0,6 | 0,1 | 0,4 | 17,7 | 5,6 | 15,5 | 4,8 | 20 |
| A19 | 0,0 5 | 1,2 | 0,8 | 0,1 | 0,4 | 17,7 | 5,6 | 15,5 | 4,8 | 20 |
| A20 | 0,0 5 | 1,4 | 0,6 | 0,1 | 0,4 | 17,7 | 5,6 | 15,5 | 4,8 | 20 |
| A21 | 0,0 5 | 1,4 | 0,6 | 0,1 | 0,4 | 17,7 | 5,6 | 15,5 | 4,8 | 20 |

- CH:: Cumolhydroperoxid
- BH:: N,N-Bis-(2-hydroxyethyl)-p-toluidin
- DM:: N,N-Dimethyl-p-toluidin
- EG:: Ethylenglykoldimethacrylat
- MA:: Methacrylamid
- MMA:: Methylmethacrylat
- PL1:: lineares Poly(methylmethacrylat-co-methylacrylat), Mw < 500.000 g/mol
- PL2:: partikuläres, unlösliches, vernetztes Polymethylmethacrylat
- ZrO₂:: Zirkoniumdioxid
- Stab:: 2,6-Di-t-butyl-4-methyl-phenol

Die Pasten B der Beispiele B1-21 wurden ebenfalls durch einfaches Vermischen der Komponenten hergestellt. Die gebildeten Pasten wurden dann über Nacht bei Raumtemperatur gelagert.

### Paste B

| | Zusammensetzung der Pasten B | | | | | |
|---|---|---|---|---|---|---|
| Beispiel-Nr. | SAC [g] | CuOct [mg] | MMA [g] | PL1 [g] | Gentamicinsulfat [g] | Stab [mg] |
| B1 | 1,0 | 25 | 21,2 | 17,5 | - | 35 |
| B2 | 1,0 | 25 | 21,2 | 17,5 | - | 35 |
| B3 | 1,0 | 40 | 21,2 | 17,5 | - | 35 |
| B4 | 1,0 | 40 | 21,2 | 17,5 | - | 35 |
| B5 | 1,0 | 45 | 21,2 | 17,5 | - | 35 |
| B6 | 1,0 | 45 | 21,2 | 17,5 | - | 35 |
| B7 | 1,0 | 45 | 21,2 | 17,5 | - | 35 |
| B8-B19 | 1,0 | 55 | 21,2 | 17,5 | -- | 35 |
| B20 | 1,0 | 55 | 21,2 | 17,5 | 1,2 | 35 |
| B21 | 1,0 | 55 | 21,2 | 17,5 | 2,4 | 35 |

- SAC:: Saccharin
- CuOct:: Kupfer(II)-2-ethylhexanoat
- MMA:: Methylmethacrylat
- PL1:: lineares Poly(methylmethacrylat-co-methylacrylat), Mw < 500.000 g/mol
- Stab:: 2,6-Di-t-butyl-4-methyl-phenol

Die Pasten A und B der Beispiele A1-21 und B1-21 wurden im Gewichtverhältnis 1:1 miteinander gemischt. Es entstanden sofort klebfreie Pasten C, die nach 4-15 Minuten aushärteten.

Mit den aus den Pasten A und B der Beispiele 1-21 hergestellten gemischten Pasten C (Gewichtsverhältnis 1 zu 1 von Paste A zu Paste B) wurden streifenförmige Prüfkörper mit den Abmessungen (75 mm × 10 mm × 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt.

| Pasten C | Pasten C | Zusammen.-setzung der Pasten C | 4-Punkt-Biegefestigkeit [MPa] | BiegeModul [MPa] | DruckFestigkeit [MPa] |
|---|---|---|---|---|---|
| P1225 | C1 | A1 + B1 | 60,1 ± 3,7 | 2346 ± 183 | 81,2 ± 6,0 |
| P1227 | C2 | A2 + B2 | 60,9 ± 3,5 | 2409 ± 113 | 92,4 ± 3,7 |
| P1232 | C3 | A3 + B3 | 62,8 ± 2,2 | 2484 ± 26 | 88,2 ± 4,0 |
| P1233 | C4 | A4 + B4 | 62,9 ± 2,6 | 2509 ± 86 | 89,7 ± 3,7 |
| P1234 | C5 | A5 + B5 | 64,7 ± 1,3 | 2593 ± 74 | 93,6 ± 3,2 |
| P1235 | C6 | A6 + B6 | 64,6 ± 1,5 | 2533 ± 25 | 94,3 ± 2,3 |
| P1236 | C7 | A7 + B7 | 60,9 ± 3,7 | 2362 ± 96 | 95,1 ± 2,0 |
| P1238 | C8 | A8 + B8 | 60,4 ± 1,5 | 2380 ± 52 | 91,5 ± 2,3 |
| P1240 | C9 | A9 + B9 | 60,2 ± 0,6 | 2407 ± 27 | 86,7 ± 4,0 |
| P1242 | C10 | A10 + B10 | 64,3 ± 1,1 | 2667 ± 103 | 100,8 ± 3,4 |
| P1244 | C11 | A11 + B11 | 60,2 ± 1,8 | 2470 ± 43 | 89,2 ± 2,9 |
| P1245 | C12 | A12 + B12 | 58,7 ± 1,3 | 2382 ± 38 | 78,1 ± 3,7 |
| P1246 | C13 | A13 + B13 | 55,0 ± 1,8 | 2186 ± 96 | 89,1 ± 4,1 |
| P1248 | C14 | A14 + B14 | 63,5 ± 0,8 | 2473 ± 26 | 88,8 ± 5,8 |
| P1249 | C15 | A15 + B15 | 57,6 ± 1,9 | 2229 ± 100 | 85,0 ± 3,0 |
| P1250 | C16 | A16 + B16 | 67,8 ± 2,6 | 2618 ± 94 | 97,6 ± 4,0 |
| P1251 | C17 | A17 + B17 | 67,1 ± 3,0 | 2969 ± 131 | 92,6 ± 3,1 |
| P1252 | C18 | A18 + B18 | 65,0 ± 1,7 | 2462 ± 67 | 91,7 ± 1,3 |
| P1253 | C19 | A19 + B19 | 67,1 ± 2,6 | 2569 ± 100 | 85,1 ± 2,6 |
| P1254 | C20 | A20 + B20 | 58,1 ± 2,2 | 2278 ±93 | 80,1 ± 2,8 |
| P1255 | C21 | A21 + B21 | 54,4 ± 2,5 | 2272 ± 68 | 80,4 ± 1,7 |

Außerdem wurden weitere Pasten B analog der Paste B 20, jedoch mit Vancomycinhydrochlorid, Clindamycinhydrochlorid, Daptomycin und Octenidindihydrochlorid anstelle von Gentamicinsulfat hergestellt. Nach Vermischung diese Pasten B mit der Paste A20 im Gewichtsverhältnis von 1 zu 1 zeigten die gemischten Pasten C ein ähnliches Aushärtungsverhalten wie die Kombination der Paste A20 mit der Paste B20 im Gewichtsverhältnis von 1 zu 1.

Zusätzlich wurden auch Pasten unter Verwendung von Bariumsulfat anstelle von Zirkoniumdioxid hergestellt. Diese Pasten hatten ein ähnliches Aushärtungsverhalten wie die aus den Pasten A1-21 und B1-21 hergestellten Pasten C.

Es wurden weiterhin auch Pasten A analog dem Beispiel A1 unter Verwendung von t-Butylhydroperoxid, t-Amyl-hydroperoxid und Dicumyl-peroxid anstelle von Cumol-hydroperoxid hergestellt. Nach Vermischung dieser Pasten A mit der Paste B1 im Gewichtsverhältnis von 1 zu 1 zeigten die gemischten Pasten ein ähnliches Verhalten wie die Kombination der Pasten A1 mit der Paste B1.

## Patentansprüche

1. Knochenzement-Kit, aufweisend eine Paste A und eine Paste B,
wobei
(a) Paste A
(a1) wenigstens ein radikalisch polymerisierbares Monomer mit einem Siedepunkt von weniger als 120°C bei einem Druck von 1.013 mbar,
(a2) als Polymerisationsinitiator wenigstens ein Peroxid, und
(a3) als Co-Polymerisationsbeschleuniger wenigstens ein tertiäres Amin, wenigstens ein Amidin oder eine Mischung aus wenigstens einem tertiärem Amin und wenigstens einem Amidin, und
(b) Paste B
(b1) wenigstens ein radikalisch polymerisierbares Monomer mit einem Siedepunkt von weniger als 120°C bei einem Druck von 1.013 mbar,
(b2) als Polymerisationsbeschleuniger wenigstens eine Schwermetallverbindung, und
(b3) als Co-Polymerisationsbeschleuniger wenigstens ein Sulfimid, wenigstens ein Dicarbonsäureimid oder eine Mischung aus wenigstens einem Sulfimid und wenigstens einem Dicarbonsäureimid,
beinhalten, wobei mindestens eine der Pasten A und B als Komponente (a4) bzw. (b4) wenigstens einen in (a1) bzw. (b1) unlöslichen Füllstoff beinhaltet.

2. Kit nach Anspruch 1, wobei das wenigstens eine radikalisch polymerisierbare Monomer (a1) bzw. (b1) ein Methacrylat-Monomer ist.

3. Kit nach Anspruch 1 oder 2, wobei die Paste A und die Paste B das wenigstens eine radikalisch polymerisierbare Monomer (a1) bzw. (b1) in einer Menge in einem Bereich von 15 bis 85 Gew.-%, jeweils bezogen auf das Gesamtgewicht der Paste A bzw. der Paste B, beinhalten.

4. Kit nach einem der vorhergehenden Ansprüche, wobei das wenigstens eine Peroxid (a2) eine Halbwertszeit > 10 Stunden bei 70°C besitzt.

5. Kit nach Anspruch 4, wobei das wenigstens eine Peroxid (a2) ausgewählt ist aus der Gruppe bestehend aus Cumol-hydroperoxid, 1,1,3,3-Tetramethylbutyl-hydroperoxid, t-Butyl-hydroperoxid, t-Amyl-hydroperoxid, Di-Isopropylbenzen-mono-hydroperoxid, Di-t-butyl-peroxid, Dicumylperoxid und t-Butylcumylperoxid.

6. Kit nach einem der vorhergehenden Ansprüche, wobei die Paste A das wenigstens eine Peroxid (a2) in einer Menge in einem Bereich von 0,01 bis 10 Gew.-%, jeweils bezogen auf das Gesamtgewicht der Paste A, beinhaltet.

7. Kit nach einem der vorhergehenden Ansprüche, wobei das wenigstens eine tertiäre Amin (a3) ausgewählt ist aus der Gruppe bestehend aus Tributylamin, Triethanolamin, N,N-Dimethyl-p-toluidin, N,N-Bis(2-hydroxy-ethyl)-p-toluidin und N,N-Dimethylanilin.

8. Kit nach einem der vorhergehenden Ansprüche, wobei das wenigstens eine Amidin (a3) ausgewählt ist aus der Gruppe 1,8-Diazabicyclo[5.4.0]undec-7-en und 1,5-Diazabicyclo(4.3.0)non-5-en.

9. Kit nach einem der vorhergehenden Ansprüche, wobei die Paste A den Co-Polymerisationsbeschleuniger (a3) in einer Menge in einem Bereich von 0,1 bis 20 Gew.-%, jeweils bezogen auf das Gesamtgewicht der Paste A, beinhaltet.

10. Kit nach einem der vorhergehenden Ansprüche, wobei die wenigstens eine Schwermetallverbindung (b2) ausgewählt ist aus der Gruppe bestehend aus Kupfer(II)-hydroxid, Kupfer(II)metharylat, Kupfer(II)acetylacetonat, Kupfer(II)-2-ethylhexanoat, Cobalt(II)hydroxid, Cobalt(II)-2-ethyl-hexanoat und basisches Kupfer(II)-carbonat.

11. Kit nach einem der vorhergehenden Ansprüche, wobei die Paste B die wenigstens eine Schwermetallverbindung (b2) in einer Menge in einem Bereich von 0,0005 bis 0,5 Gew.-%, bezogen auf das Gesamtgewicht der Paste B, beinhaltet.

12. Kit nach einem der vorhergehenden Ansprüche, wobei das wenigstens eine Sulfimid (b3) Saccharin ist.

13. Kit nach einem der vorhergehenden Ansprüche, wobei das wenigstens eine Dicarbonsäureimid (b3) Phthalimid, Maleimid oder Succinimid ist.

14. Kit nach einem der vorhergehenden Ansprüche, wobei die Paste B den Co-Polymerisationsbeschleuniger (b3) in einer Menge in einem Bereich von 0,1 bis 10 Gew.-%, bezogen auf das Gesamtgewicht der B, beinhaltet.

15. Kit nach einem der vorhergehenden Ansprüche, wobei der wenigstens eine in (a1) bzw. (b1) unlösliche Füllstoff (a4) bzw. (b4) ein partikuläres Polymer ist.

16. Kit nach einem der vorhergehenden Ansprüche, wobei der wenigstens eine in (a1) bzw. (b1) unlösliche Füllstoff (a4) bzw. (b4) ein unlösliches Polymer ausgewählt aus der Gruppe bestehend aus vernetztem Poly(methylmethacrylat-co-methylacrylat), vernetztem Poly(methylmetha-crylat) und einer Mischung dieser beiden Polymere ist.

17. Kit nach einem der vorhergehenden Ansprüche, wobei Paste A, Paste B oder Paste A und Paste B ein in (a1) bzw. (b1) lösliches Polymer (a5) bzw. (b5) beinhalten.

18. Kit nach einem der vorhergehenden Ansprüche, wobei das in (a1) bzw. (b1) lösliche Polymer (a5) bzw. (b5) ausgewählt ist aus der Gruppe bestehend aus Poly(methacrylsäure-methylester), Poly(methacrylsäure-ethylester), Poly-(methylmethacrylsäurepropylester), Poly(methacryl-säureisopropylester), Poly(methylmeth-acrylat-co-methylacrylat) und Poly(styrol-co-methyl-methacrylat).

19. Verwendung eines Kits nach einem der vorhergehenden Ansprüche zur Herstellung einer Knochenzement-Paste zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.

20. Formkörper, erhalten durch die Polymerisation einer Paste, erhältlich durch das Vermischen der Paste A und der Paste B des Knochenzement-Kits, wie in einem der Ansprüche 1 bis 18 definiert.

## Claims

1. Bone cement kit, comprising a paste A and a paste B, whereby
(a) paste A contains
(a1) at least one monomer for radical polymerisation having a boiling point of less than 120°C at a pressure of 1,013 mbar,
(a2) as polymerisation initiator, at least one peroxide, and
(a3) as polymerisation co-accelerator, at least one tertiary amine, at least one amidine or a mixture of at least one tertiary amine and at least one amidine, and
(b) paste B contains
(b1) at least one monomer for radical polymerisation having a boiling point of less than 120°C at a pressure of 1.013 mbar,
(b2) as polymerisation accelerator, at least one heavy metal compound, and
(b3) as polymerisation co-accelerator at least one sulfimide, at least one dicarboxylic acid imide or a mixture of at least one sulfimide and at least one dicarboxylic acid imide,
whereby at least one of the pastes A and B contains, as component (a4) and/or (b4), at least one filling agent that is insoluble in (a1) and/or (b1).

2. Kit according to claim 1, whereby the at least one monomer for radical polymerisation (a1) and/or (b1) is a methacrylate monomer.

3. Kit according to claim 1 or 2, whereby paste A and paste B contain an amount of the at least one monomer for radical polymerisation (a1) and/or (b1) in the range of 15 to 85 % by weight, each relative to the total weight of paste A and/or paste B.

4. Kit according to any one of the preceding claims, whereby the at least one peroxide (a2) has a half-life > 10 hours at 70°C.

5. Kit according to claim 4, whereby the at least one peroxide (a2) is selected from the group consisting of cumene-hydroperoxide, 1,1,3,3-tetramethylbutyl-hydroperoxide, t-butyl-hydroperoxide, t-amyl-hydroperoxide, diisopropylbenzen-mono-hydroperoxide, di-t-butyl-peroxide, dicumylperoxide, and t-butylcumylperoxide.

6. Kit according to any one of the preceding claims, whereby paste A contains an amount of the at least one peroxide (a2) in the range of 0.01 to 10 % by weight, each relative to the total weight of paste A.

7. Kit according to any one of the preceding claims, whereby the at least one tertiary amine (a3) is selected from the group consisting of tributylamine, triethanolamine, N,N-dimethyl-p-toluidine, N,N-bis(2-hydroxy-ethyl)-p-toluidine, and N,N-dimethyl-aniline.

8. Kit according to any one of the preceding claims, whereby the at least one amidine (a3) is selected from the group of 1,8-diazabicyclo[5.4.0]undec-7-ene and 1,5-diazabicyclo[4.3.0]on-5-ene.

9. Kit according to any one of the preceding claims, whereby paste A contains an amount of the polymerisation co-accelerator (a3) in the range of 0.1 to 20 % by weight, each relative to the total weight of paste A.

10. Kit according to any one of the preceding claims, whereby the at least one heavy metal compound (b2) is selected from the group consisting of copper(II) hydroxide, copper(II) metharylate, copper(II) acetylacetonate, copper(II) 2-ethylhexanoate, cobalt(II) hydroxide, cobalt(II) 2-ethylhexanoate, and basic copper(II) carbonate.

11. Kit according to any one of the preceding claims, whereby paste B contains an amount of the at least one heavy metal compound (b2) in the range of 0.0005 to 0.5 % by weight, relative to the total weight of paste B.

12. Kit according to any one of the preceding claims, whereby the at least one sulfimide (b3) is saccharine.

13. Kit according to any one of the preceding claims, whereby the at least one dicarboxylic acid imide (b3) is phthalimide, maleimide or succinimide.

14. Kit according to any one of the preceding claims, whereby paste B contains an amount of the polymerisation co-accelerator (b3) in the range of 0.1 to 10 % by weight, relative to the total weight of paste B.

15. Kit according to any one of the preceding claims, whereby the at least one filling agent (a4) and/or (b4) that is insoluble in (a1) and/or (b1) is a particulate polymer.

16. Kit according to any one of the preceding claims, whereby the at least one filling agent (a4) and/or (b4) that is insoluble in (a1) and/or (b1) is an insoluble polymer selected from the group consisting of cross-linked poly(methylmethacrylate-co-methylacrylate), cross-linked poly(methylmethacrylate), and a mixture of said two polymers.

17. Kit according to any one of the preceding claims, whereby paste A, paste B or paste A and paste B contain a polymer (a5) and/or (b5) that is soluble in (a1) and/or (b1).

18. Kit according to any one of the preceding claims, whereby the polymer (a5) and/or (b5) that is soluble in (a1) and/or (b1) is selected from the group consisting of poly(methacrylic acid methylester), poly(methacrylic acid ethylester), poly(methylmethacrylic acid propylester), poly(methacrylic acid isopropylester), poly(methylmethacrylate-co-methacrylate), and poly(styrene-co-methyl-methacrylate).

19. Use of a kit according to any one of the preceding claims for producing a bone cement paste for mechanical fixation of articular endoprostheses, for covering skull defects, for filling bone cavities, for femuroplasty, for vertebroplasty, for kyphoplasty, for the manufacture of spacers, and for the production of carrier materials for local antibiotics therapy.

20. Form body, obtained by polymerisation of a paste, obtainable by mixing paste A and paste B of the bone cement kit, as defined in any one of the claims 1 to 18.

## Revendications

1. Kit de ciment osseux présentant une pâte A et une pâte B,
dans lequel
(a) la pâte A contient
(a1) au moins un monomère polymérisable par voie radicalaire avec un point d'ébullition inférieur à 120°C à une pression de 1,013 mbar,
(a2) au moins un peroxyde en tant qu'initiateur de polymérisation, et
(a3) au moins une amine tertiaire, au moins une amidine ou un mélange d'au moins une amine tertiaire et d'au moins une amidine en tant qu'accélérateur de copolymérisation, et
(b) la pâte B contient
(b1) au moins un monomère polymérisable par voie radicalaire avec un point d'ébullition inférieur à 120°C à une pression de 1,013 mbar,
(b2) au moins un composé de métal lourd en tant qu'accélérateur de polymérisation, et
(b3) au moins un sulfimide, au moins un imide d'acide dicarboxylique ou un mélange d'au moins un sulfimide et d'au moins un imide d'acide dicarboxylique en tant qu'accélérateur de copolymérisation,
dans lequel au moins une des pâtes A et B contient en tant que composant (a4) ou (b4) au moins une charge insoluble dans (a1) ou (b1).

2. Kit selon la revendication 1, dans lequel l'au moins un monomère polymérisable par voie radicalaire (a1) ou (b1) est un monomère de méthacrylate.

3. Kit selon la revendication 1 ou 2, dans lequel la pâte A et la pâte B contiennent l'au moins un monomère polymérisable par voie radicalaire (a1) ou (b1) en une quantité dans une plage de 15 à 85 % en poids, à chaque fois par rapport au poids total de la pâte A ou de la pâte B.

4. Kit selon une des revendications précédentes, dans lequel l'au moins un peroxyde (a2) possède une demi-vie > 10 heures à 70°C.

5. Kit selon la revendication 4, dans lequel l'au moins un peroxyde (a2) est sélectionné parmi le groupe constitué de l'hydroperoxyde de cumène, l'hydroperoxyde de 1,1,3,3-tétraméthylbutyle, l'hydroperoxyde de t-butyle, l'hydroperoxyde de t-amyle, le mono-hydroperoxyde de di-isopropylbenzène, le peroxyde de di-t-butyle, le peroxyde de dicumyle et le peroxyde de t-butylcumyle.

6. Kit selon une des revendications précédentes, dans lequel la pâte A contient l'au moins un peroxyde (a2) en une quantité dans une plage de 0,01 à 10 % en poids, à chaque fois par rapport au poids total de la pâte A.

7. Kit selon une des revendications précédentes, dans lequel l'au moins une amine tertiaire (a3) est sélectionnée parmi le groupe constitué de la tributylamine, la triéthanolamine, la N,N-diméthyl-p-toluidine, la N,N-bis(2-hydroxy-éthyl)-p-toluidine et la N,N-diméthyl-aniline.

8. Kit selon une des revendications précédentes, dans lequel l'au moins une amidine (a3) est sélectionnée parmi le groupe du 1,8-diazabicyclo[5.4.0]undéc-7-ène et du 1,5-diazabicyclo[4.3.0]non-5-ène.

9. Kit selon une des revendications précédentes, dans lequel la pâte A contient l'accélérateur de copolymérisation (a3) en une quantité dans une plage de 0,1 à 20 % en poids, à chaque fois par rapport au poids total de la pâte A.

10. Kit selon une des revendications précédentes, dans lequel l'au moins un composé de métal lourd (b2) est sélectionné parmi le groupe constitué de l'hydroxyde de cuivre(II), le méthacrylate de cuivre(II), l'acétylacétonate de cuivre(II), le 2-éthylhexanoate de cuivre(II), l'hydroxyde de cobalt(II), le 2-éthyl-hexanoate de cobalt(II) et le carbonate de cuivre(II) basique.

11. Kit selon une des revendications précédentes, dans lequel la pâte B contient l'au moins un composé de métal lourd (b2) en une quantité dans une plage de 0,0005 à 0,5 % en poids, par rapport au poids total de la pâte B.

12. Kit selon une des revendications précédentes, dans lequel l'au moins un sulfimide (b3) est une saccharine.

13. Kit selon une des revendications précédentes, dans lequel l'au moins un imide d'acide dicarboxylique (b3) est un phtalimide, un maléimide ou un succinimide.

14. Kit selon une des revendications précédentes, dans lequel la pâte B contient l'accélérateur de copolymérisation (b3) en une quantité dans une plage de 0,1 à 10 % en poids, par rapport au poids total de la pâte B.

15. Kit selon une des revendications précédentes, dans lequel l'au moins une charge (a4) ou (b4) insoluble dans (a1) ou (b1) est un polymère particulaire.

16. Kit selon une des revendications précédentes, dans lequel l'au moins une charge (a4) ou (b4) insoluble dans (a1) ou (b1) est un polymère insoluble sélectionné parmi le groupe constitué de poly(méthylméthacrylate-co-méthylacrylate) réticulé, poly(méthylmétha-crylate) réticulé et d'un mélange de ces deux polymères.

17. Kit selon une des revendications précédentes, dans lequel la pâte A, la pâte B ou la pâte A et la pâte B contiennent un polymère (a5) ou (b5) soluble dans (a1) ou (b1).

18. Kit selon une des revendications précédentes, dans lequel le polymère (a5) ou (b5) soluble dans (a1) ou (b1) est sélectionné parmi le groupe constitué de l'ester méthylique d'acide poly(méthacrylique), l'ester éthylique d'acide poly(méthacrylique), l'ester propylique d'acide poly(méthylméthacrylique), l'ester propylique d'acide poly(méthacrylique), le poly(méthylméth-acrylate-co-méthylacrylate) et le poly(styrène-co-méthyl-méthacrylate).

19. Utilisation d'un kit selon une des revendications précédentes pour la fabrication d'une pâte de ciment osseux pour la fixation mécanique de prothèses articulées, pour le recouvrement de défauts crâniens, pour le remplissage de cavités osseuses, pour la fémoroplastie, pour la vertébroplastie, pour la kyphoplastie, pour la fabrication d'écarteurs ou pour la fabrication de matériaux de support pour l'antibiothérapie locale.

20. Corps moulé obtenu par la polymérisation d'une pâte, pouvant être obtenue par le mélange de la pâte A et de la pâte B du kit de ciment osseux, comme défini dans une des revendications 1 à 18.
